# EUROPEAN PATENT SPECIFICATION

(11) **EP 3 019 171 B1**
(45) Date of publication and mention of the grant of the patent: **05.09.2018**
(21) Application number: 14823386.9
(22) Date of filing: 07.07.2014
(51) Int. Cl.: A61K 31/519, A61K 31/497, C07D 487/04, A61P 29/00, A61K 45/06

(54) **SUBSTITUTED AMIDOPYRAZOLE INHIBITORS OF INTERLEUKIN RECEPTOR-ASSOCIATED KINASES (IRAK-4)**
SUBSTITUIERTE AMIDOPYRAZOLHEMMER VON INTERLEUKINREZEPTOR-ASSOZIIERTEN KINASEN (IRAK-4)
INHIBITEURS AMIDOPYRAZOLE SUBSTITUÉS DE KINASES ASSOCIÉES AUX RÉCEPTEURS DE L'INTERLEUKINE (IRAK -4)

(30) Priority: 11.07.2013 US 201361845082 P
(43) Date of publication of application: 18.05.2016
(73) Proprietor: Merck Sharp & Dohme Corp., Rahway, NJ 07065-0907 (US)
(72) Inventor: LIM, Jongwon, Boston, Massachusetts 02115-5727 (US); ALTMAN, Michael, D., Boston, Massachusetts 02115-5727 (US)
(74) Representative: Jaap, David Robert
(86) International application number: PCT/US2014/045517
(87) International publication number: WO 2015/006181

(56) References cited:
- WO-A1-2012/129258
- WO-A1-2012/129258
- US-A1- 2012 015 962
- ZHULUN WANG ET AL: "IRAK-4 Inhibitors for Inflammation", CURRENT TOPICS IN MEDICINAL CHEMISTRY, vol. 9, no. 8, 1 May 2009 (2009-05-01), pages 724-737, XP055256417, HILVERSUM; NL ISSN: 1568-0266, DOI: 10.2174/156802609789044407
- BUCKLEY G M ET AL: "IRAK-4 inhibitors. Part 1: A series of amides", BIOORGANIC & MEDICINAL CHEMISTRY LETTERS, PERGAMON, AMSTERDAM, NL, vol. 18, no. 11, 1 June 2008 (2008-06-01), pages 3211-3214, XP022711199, ISSN: 0960-894X, DOI: 10.1016/J.BMCL.2008.04.058 [retrieved on 2008-04-26]

## Description

### BACKGROUND OF THE INVENTION

This invention relates to amidopyrazole compounds that are inhibitors of Interleukin receptor-associated kinases, in particular IRAK-4, and are useful in the treatment of cellular proliferative diseases, for example, cancer, hyperplasia, restenosis, cardiac hypertrophy, immune disorders and inflammation.

Interleukin-1 receptor-associated kinases (IRAKs) are key components in the signal transduction pathways utilized by interleukin-1 receptor (IL-1R), interleukin-18 receptor (IL-18R), and Toll-like receptors (TLRs). Since TLRs initiate the first-wave of inflammatory signals and innate immune responses, they play a key role in many disease processes, including response to infections and auto-inflammatory disorders.

IRAK-4 belongs to a family of mammalian IRAKs that include IRAK-1, IRAK-2 and IRAK-M (also known as IRAK-3). IRAK-4 shares the domain structure of the other IRAKs and it is able to activate similar signal transduction pathways, namely NF-κB and MAPK pathways. It rapidly and transiently associates with IRAK-1 and TRAF6 in an IL-1-dependent manner but it is not functionally redundant with IRAK-1. Most strikingly, IRAK-4 is an active protein kinase and requires its kinase activity to activate NF-κB. Additionally, IRAK-4 might act upstream of IRAK-1 as an IRAK-1 activator. See Li, S. et al., "IRAK-4: a novel member of the IRAK family with the properties of an IRAK-kinase," Proc Natl Acad Sci U S A. 2002 Apr 16;99(8):5567-72.

All four IRAK family members appear to play a role in Toll and IL-1R signaling. However, out of four members in the mammalian IRAK family, IRAK-4 is considered to be the "master IRAK", the only family member indispensable for IL-1R/TLR signaling. Mouse knockout studies have demonstrated the essential role for IRAK-4 in IL-1R, IL-18R and most TLR signaling (see, Suzuki, N, et al, "Severe impairment of interleukin-1 and Toll-like receptor signaling in mice lacking IRAK-4," Nature, 2002, 416, 750-756). Furthermore, knock-in experiments by several groups have clearly demonstrated that IRAK-4 requires its kinase activity for its function.

In humans, mutations resulting in IRAK-4 deficiency have been linked to susceptibility to bacterial infections, especially recurrent pyogenic bacterial infections (see, Picard, C., et al. "Pyogenic bacterial infections in humans with IRAK-4 deficiency." Science, 2003, 299, 2076-2079). While IRAK-4 deficient children are susceptible to certain pyogenic infections, adults are not prone to chronic infections. It is possible that protective immunity remains sufficiently preserved to protect against infection while modulation of IRAK-4 function through kinase inhibition may tone down inflammatory response.

Given the critical role of IRAK-4 in inflammatory processes, modulation of IRAK-4 kinase activity presents an attractive therapeutic approach for the treatment of immune and inflammatory diseases. The recent success in the determination of the 3-dimensional structure of the IRAK-4 kinase domain in complex with inhibitors has facilitated the understanding of the mechanistic role of IRAK-4 in immunity and inflammation as well as the development of specific IRAK-4 kinase inhibitors. See, Wang, et al, "IRAK-4 Inhibitors for Inflammation," Current Topics in Medicinal Chemistry, 2009, 9, 724-737.

It would be useful to develop potent IRAK-4 inhibitors with desired properties as new anti-inflammatory and anti-neoplastic therapeutic agents.

A number of studies have established the relationship between activation of the NF-κB and the progression of cancer. Since TLRs are prominent activators of the NF-κB pathway, it has been hypothesized that stimulation of TLRs is a factor in the development of human cancers. See, Chen, et al, "Inflammation, Cancer, and Chemoresistance: Taking Advantage of the Toll-Like Receptor Signaling Pathway," American Journal of Reproductive Immunology, 2007, 57 (2), 93-107. Given the central role of IRAK-4 in the TLR driven inflammation pathway, it has been proposed that inhibition of IRAK-4 may lead to a new class of anticancer agents. See, Shaw, et al, "Characterization of Novel Diaryl Oxazole-Based Compounds as Potential Agents to Treat Pancreatic Cancer," Journal of Pharmacology and Experimental Therapeutics, 2009, 331 (2), 636-647.

Inhibitors of Interleukin receptor-associated kinases are known, see WO2008/030584, WO2012/007375, WO2012/097013, WO2011/043371, WO2003/030902, WO2011/006567.

Amidopyrazole compounds that are inhibitors of Interleukin receptor-associated kinases, in particular IRAK-4, are disclosed in WO2012/129258.

There exists a need in the art for small molecule compounds having desirable physiochemical properties that are useful for treating cellular proliferative diseases.

### SUMMARY OF THE INVENTION

The present invention relates to novel substituted amidopyrazole compounds that are inhibitors of Interleukin receptor-associated kinases, in particular IRAK-4, and are useful in the treatment of cellular proliferative diseases.

### DETAILED DESCRIPTION OF THE INVENTION

The compounds of this invention are useful in the inhibition of Interleukin receptor-associated kinases, in particular IRAK-4, and are:
*N*-[3-{4-[(3*R*)-1,1-dioxidotetrahydrothiophen-3-yl]piperazin-1-yl}-1-(5-methylpyridin-2-yl)-1*H*-pyrazol-5-yl]pyrazolo[1,5-*a*]pyrimidine-3-carboxamide (**8**);
*N*-[3-{4-[(3*S*)-1,1-dioxidotetrahydrothiophen-3-yl]piperazin-1-yl}-1-(5-methylpyridin-2-yl)-1*H-*pyrazol-5-yl]pyrazolo[1,5-*a*]pyrimidine-3-carboxamide (**9**); and
*N*-{3-[4-(2-amino-2-oxoethyl)piperazin-1-yl]-1-(5-methylpyridin-2-yl)-1*H*-pyrazol-5-yl}pyrazolo[1,5-*a*]pyrimidine-3-carboxamide (**16**);
or a pharmaceutically acceptable salt or stereoisomer thereof.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention. In addition, the compounds disclosed herein may exist as tautomers and both tautomeric forms are intended to be encompassed by the scope of the invention, even though only one tautomeric structure is depicted.

In the compounds, the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds. For example, different isotopic forms of hydrogen (H) include protium (1H) and deuterium (2H). Protium is the predominant hydrogen isotope found in nature. Enriching for deuterium may afford certain therapeutic advantages, such as increasing in vivo half-life or reducing dosage requirements, or may provide a compound useful as a standard for characterization of biological samples. Isotopically-enriched compounds disclosed can be prepared without undue experimentation by conventional techniques well known to those skilled in the art or by processes analogous to those described in the Schemes and Examples herein using appropriate isotopically-enriched reagents and/or intermediates.

Included in the instant invention is the free form of compounds of the instant invention, as well as the pharmaceutically acceptable salts and stereoisomers thereof. The term "free form" refers to the amine compounds in non-salt form. The encompassed pharmaceutically acceptable salts not only include the salts exemplified for the specific compounds described herein, but also all the typical pharmaceutically acceptable salts of the free form of compounds of the instant invention. The free form of the specific salt compounds described may be isolated using techniques known in the art. For example, the free form may be regenerated by treating the salt with a suitable dilute aqueous base solution such as dilute aqueous NaOH, potassium carbonate, ammonia and sodium bicarbonate. The free forms may differ from their respective salt forms somewhat in certain physical properties, such as solubility in polar solvents, but the acid and base salts are otherwise pharmaceutically equivalent to their respective free forms for purposes of the invention.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reacting the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base.

Thus, pharmaceutically acceptable salts of the compounds of this invention include the conventional non-toxic salts of the compounds of this invention as formed by reacting a basic instant compound with an inorganic or organic acid. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like.

When the compound of the present invention is acidic, suitable "pharmaceutically acceptable salts" refers to salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine caffeine, choline, N,N¹-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like. When the compound of the present invention is acidic, the term "free form" refers to the compound in its non-salt form, such that the acidic functionality is still protonated.

The preparation of the pharmaceutically acceptable salts described above and other typical pharmaceutically acceptable salts is more fully described by Berg et al., "Pharmaceutical Salts," J. Pharm. Sci., 1977:66:1-19.

It will also be noted that the compounds of the present invention may potentially be internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom. An isolated compound having internally balanced charges, and thus not associated with an intermolecular counterion, may also be considered the "free form" of a compound.

### UTILITY

According to yet another embodiment, the present invention provides a compound as described above for use in treating or reducing the severity of a disease in a patient, wherein said disease is selected from IRAK4 mediated pathologies, such as rheumatoid arthritis, multiple sclerosis, sepsis, osteoarthritis, inflammatory bowel disease, Parkinson's disease, cardiac contractile dysfunction, type I diabetes, type II diabetes or familial cold autoinflammatory syndrome, allergic disease, cancer, psoriasis, asthma or graft rejection.

The compounds of the invention find use in a variety of applications. As will be appreciated by those skilled in the art, the kinase activity of IRAK-4 may be modulated in a variety of ways; that is, one can affect the phosphorylation/activation of IRAK-4 either by modulating the initial phosphorylation of the protein or by modulating the autophosphorylation of the other active sites of the protein. Alternatively, the kinase activity of IRAK-4 may be modulated by affecting the binding of a substrate of IRAK-4 phosphorylation.

The compounds of the invention are used to treat or prevent inflammation related diseases. Disease states which can be treated by the compounds and compositions provided herein include, cancer, autoimmune disease, viral disease, fungal disease, neurological/neurodegenerative disorders, arthritis, inflammation, anti-proliferative (e.g. ocular retinopathy), neuronal, alopecia, cardiovascular disease, graft rejection, inflammatory bowel disease, proliferation induced after medical procedures, including, but not limited to, surgery, angioplasty, and the like. It is appreciated that in some cases the cells may not be in a hyper- or hypoproliferation state (abnormal state) and still require treatment. Thus, in one embodiment, the invention herein includes application to cells or individuals which are afflicted or may eventually become afflicted with any one of these disorders or states.

The compounds of this invention may be administered to mammals, including humans, either alone or, in combination with pharmaceutically acceptable carriers, excipients or diluents, in a pharmaceutical composition, according to standard pharmaceutical practice. The compounds can be administered orally or parenterally, including the intravenous, intramuscular, intraperitoneal, subcutaneous and topical routes of administration.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water soluble taste masking material such as hydroxypropylmethyl-cellulose or hydroxypropylcellulose, or a time delay material such as ethyl cellulose, cellulose acetate buryrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethylene-oxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsion. The oily phase may be a vegetable oil, for example olive oil or arachis oil, or a mineral oil, for example liquid paraffin or mixtures of these. Suitable emulsifying agents may be naturally-occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavouring agents, preservatives and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of sterile injectable aqueous solutions. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution then introduced into a water and glycerol mixture and processed to form a microemulsion.

The injectable solutions or microemulsions may be introduced into a patient's bloodstream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS™ model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

For topical use, creams, ointments, jellies, solutions or suspensions, etc., containing the compound of the instant invention are employed. (For purposes of this application, topical application shall include mouth washes and gargles.)

The compounds for the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol.

When a composition according to this invention is administered into a human subject, the daily dosage will normally be determined by the prescribing physician with the dosage generally varying according to the age, weight, and response of the individual patient, as well as the severity of the patient's symptoms.

The dosage regimen utilizing the compounds of the instant invention can be selected in accordance with a variety of factors including type, age, weight, sex; the route of administration; the renal and hepatic function of the patient; and the particular compound or salt thereof employed. An ordinarily skilled physician or veterinarian can readily determine and prescribe the effective amount of the drug required to treat, for example, to prevent, inhibit (fully or partially) or arrest the progress of the disease. For example, compounds of the instant invention can be administered in a total daily dose of up to 10,000 mg. Compounds of the instant invention can be administered once daily (QD), or divided into multiple daily doses such as twice daily (BID), and three times daily (TID). Compounds of the instant invention can be administered at a total daily dosage of up to 10,000 mg, e.g., 2,000 mg, 3,000 mg, 4,000 mg, 6,000 mg, 8,000 mg or 10,000 mg, which can be administered in one daily dose or can be divided into multiple daily doses as described above.

For example, compounds of the instant invention can be administered in a total daily dose of up to 1,000 mg. Compounds of the instant invention can be administered once daily (QD), or divided into multiple daily doses such as twice daily (BID), and three times daily (TID). Compounds of the instant invention can be administered at a total daily dosage of up to 1,000 mg, e.g., 200 mg, 300 mg, 400 mg, 600 mg, 800 mg or 1,000 mg, which can be administered in one daily dose or can be divided into multiple daily doses as described above.

In addition, the administration can be continuous, i.e., every day, or intermittently. The terms "intermittent" or "intermittently" as used herein means stopping and starting at either regular or irregular intervals. For example, intermittent administration of a compound of the instant invention may be administration one to six days per week or it may mean administration in cycles (e.g. daily administration for two to eight consecutive weeks, then a rest period with no administration for up to one week) or it may mean administration on alternate days.

In addition, the compounds of the instant invention may be administered according to any of the schedules described above, consecutively for a few weeks, followed by a rest period. For example, the compounds of the instant invention may be administered according to any one of the schedules described above from two to eight weeks, followed by a rest period of one week, or twice daily at a dose of 100 - 500 mg for three to five days a week. In another particular embodiment, the compounds of the instant invention may be administered three times daily for two consecutive weeks, followed by one week of rest.

Any one or more of the specific dosages and dosage schedules of the compounds of the instant invention, may also be applicable to any one or more of the therapeutic agents to be used in the combination treatment (hereinafter refered to as the "second therapeutic agent").

Moreover, the specific dosage and dosage schedule of this second therapeutic agent can further vary, and the optimal dose, dosing schedule and route of administration will be determined based upon the specific second therapeutic agent that is being used.

Of course, the route of administration of the compounds of the instant invention is independent of the route of administration of the second therapeutic agent. In an embodiment, the administration for a compound of the instant invention is oral administration. In another embodiment, the administration for a compound of the instant invention is intravenous administration. Thus, in accordance with these embodiments, a compound of the instant invention is administered orally or intravenously, and the second therapeutic agent can be administered orally, parenterally, intraperitoneally, intravenously, intraarterially, transdermally, sublingually, intramuscularly, rectally, transbuccally, intranasally, liposomally, via inhalation, vaginally, intraoccularly, via local delivery by catheter or stent, subcutaneously, intraadiposally, intraarticularly, intrathecally, or in a slow release dosage form.

In addition, a compound of the instant invention and second therapeutic agent may be administered by the same mode of administration, i.e. both agents administered e.g. orally, by IV. However, it is also within the scope of the present invention to administer a compound of the instant invention by one mode of administration, e.g. oral, and to administer the second therapeutic agent by another mode of administration, e.g. IV or any other ones of the administration modes described hereinabove.

The first treatment procedure, administration of a compound of the instant invention, can take place prior to the second treatment procedure, i.e., the second therapeutic agent, after the treatment with the second therapeutic agent, at the same time as the treatment with the second therapeutic agent, or a combination thereof. For example, a total treatment period can be decided for a compound of the instant invention. The second therapeutic agent can be administered prior to onset of treatment with a compound of the instant invention or following treatment with a compound of the instant invention.

The instant compounds are also useful in combination with other therapeutic agents. Combinations of the presently disclosed compounds with therapeutic agents are within the scope of the invention. A person of ordinary skill in the art would be able to discern which combinations of agents would be useful based on the particular characteristics of the drugs and the pathologies involved. The instant compounds are also useful in combination with known therapeutic agents.

The instant compounds are useful in combination with a known anti-inflammatory agent. In one embodiment, the anti-inflammatory agent is a nonsteroidal anti-inflammatory drug (NSAID). In one embodiment, the NSAID is selected from the group consisting of salicylates, indomethacin, flurbiprofen, diclofenac, ketorolac, naproxen, piroxicam, tebufelone, ibuprofen, etodolac, nabumetone, tenidap, alcofenac, antipyrine, aminopyrine, dipyrone, aminopyrone, phenylbutazone, clofezone, oxyphenbutazone, prenazone, apazone, benzydamine, bucolome, cinchophen, clonixin, ditrazol, epirizole, fenoprofen, floctafenin, flufenamic acid, glaphenine, indoprofen, ketoprofen, loxoprofen, meclofenamic acid, mefenamic acid, niflumic acid, phenacetin, salidifamides, sulindac, suprofen, tolmetin, a pharmaceutically acceptable salt thereof, and a mixture thereof.

In another embodiment, the NSAID is a selective COX-2 inhibitor. For purposes of this specification NSAID's which are selective inhibitors of COX-2 are defined as those which possess a specificity for inhibiting COX-2 over COX-1 of at least 100 fold as measured by the ratio of IC₅₀ for COX-2 over IC₅₀ for COX-1 evaluated by cell or microsomal assays. Such compounds include, those disclosed in U.S. Pat. 5,474,995, U.S. Pat. 5,861,419, U.S. Pat. 6,001,843, U.S. Pat. 6,020,343, U.S. Pat. 5,409,944, U.S. Pat. 5,436,265, U.S. Pat. 5,536,752, U.S. Pat. 5,550,142, U.S. Pat. 5,604,260, U.S. 5,698,584, U.S. Pat. 5,710,140, WO 94/15932, U.S. Pat. 5,344,991, U.S. Pat. 5,134,142, U.S. Pat. 5,380,738, U.S. Pat. 5,393,790, U.S. Pat. 5,466,823, U.S. Pat. 5,633,272, and U.S. Pat. 5,932,598.

Compounds that have been described as specific inhibitors of COX-2 and are therefore useful in the present invention include: parecoxib, CELEBREX® and BEXTRA® or a pharmaceutically acceptable salt thereof.

The instant compounds are useful in combination with a known anti-cancer agent. Combinations of the presently disclosed compounds with anti-cancer agents are within the scope of the invention. Examples of such anti-cancer agents can be found in Cancer Principles and Practice of Oncology by V.T. Devita and S. Hellman (editors), 6th edition (February 15, 2001), Lippincott Williams & Wilkins Publishers. A person of ordinary skill in the art would be able to discern which combinations of agents would be useful based on the particular characteristics of the drugs and the cancer involved. Such agents include the following: estrogen receptor modulators, androgen receptor modulators, retinoid receptor modulators, cytotoxic/cytostatic agents, antiproliferative agents, prenyl-protein transferase inhibitors, HMG-CoA reductase inhibitors and other angiogenesis inhibitors, HIV protease inhibitors, reverse transcriptase inhibitors, inhibitors of cell proliferation and survival signaling, bisphosphonates, aromatase inhibitors, siRNA therapeutics, γ-secretase inhibitors, agents that interfere with receptor tyrosine kinases (RTKs) and agents that interfere with cell cycle checkpoints.

In one embodiment, the anti-cancer agent is selected from the group consisting of abarelix (Plenaxis depot®); aldesleukin (Prokine®); Aldesleukin (Proleukin®); Alemtuzumabb (Campath®); alitretinoin (Panretin®); allopurinol (Zyloprim®); altretamine (Hexalen®); amifostine (Ethyol®); anastrozole (Arimidex®); arsenic trioxide (Trisenox®); asparaginase (Elspar®); azacitidine (Vidaza®); bevacuzimab (Avastin®); bexarotene capsules (Targretin®); bexarotene gel (Targretin®); bleomycin (Blenoxane®); bortezomib (Velcade®); busulfan intravenous (Busulfex®); busulfan oral (Myleran®); calusterone (Methosarb®); capecitabine (Xeloda®); carboplatin (Paraplatin®); carmustine (BCNU®, BiCNU®); carmustine (Gliadel®); carmustine with Polifeprosan 20 Implant (Gliadel Wafer®); celecoxib (Celebrex®); cetuximab (Erbitux®); chlorambucil (Leukeran®); cisplatin (Platinol®); cladribine (Leustatin®, 2-CdA®); clofarabine (Clolar®); cyclophosphamide (Cytoxan®, Neosar®); cyclophosphamide (Cytoxan Injection®); cyclophosphamide (Cytoxan Tablet®); cytarabine (Cytosar-U®); cytarabine liposomal (DepoCyt®); dacarbazine (DTIC-Dome®); dactinomycin, actinomycin D (Cosmegen®); Darbepoetin alfa (Aranesp®); daunorubicin liposomal (DanuoXome®); daunorubicin, daunomycin (Daunorubicin®); daunorubicin, daunomycin (Cerubidine®); Denileukin diftitox (Ontak®); dexrazoxane (Zinecard®); docetaxel (Taxotere®); doxorubicin (Adriamycin PFS®); doxorubicin (Adriamycin®, Rubex®); doxorubicin (Adriamycin PFS Injection®); doxorubicin liposomal (Doxil®); dromostanolone propionate (dromostanolone®); dromostanolone propionate (masterone injection®); Elliott's B Solution (Elliott's B Solution®); epirubicin (Ellence®); Epoetin alfa (epogen®); erlotinib (Tarceva®); estramustine (Emcyt®); etoposide phosphate (Etopophos®); etoposide, VP-16 (Vepesid®); exemestane (Aromasin®); Filgrastim (Neupogen®); floxuridine (intraarterial) (FUDR®); fludarabine (Fludara®); fluorouracil, 5-FU (Adrucil®); fulvestrant (Faslodex®); gefitinib (Iressa®); gemcitabine (Gemzar®); gemtuzumab ozogamicin (Mylotarg®); goserelin acetate (Zoladex Implant®); goserelin acetate (Zoladex®); histrelin acetate (Histrelin implant®); hydroxyurea (Hydrea®); Ibritumomab Tiuxetan (Zevalin®); idarubicin (Idamycin®); ifosfamide (IFEX®); imatinib mesylate (Gleevec®); interferon alfa 2a (Roferon A®); Interferon alfa-2b (Intron A®); irinotecan (Camptosar®); lenalidomide (Revlimid®); letrozole (Femara®); leucovorin (Wellcovorin®, Leucovorin®); Leuprolide Acetate (Eligard®); levamisole (Ergamisol®); lomustine, CCNU (CeeBU®); meclorethamine, nitrogen mustard (Mustargen®); megestrol acetate (Megace®); melphalan, L-PAM (Alkeran®); mercaptopurine, 6-MP (Purinethol®); mesna (Mesnex®); mesna (Mesnex tabs®); methotrexate (Methotrexate®); methoxsalen (Uvadex®); mitomycin C (Mutamycin®); mitotane (Lysodren®); mitoxantrone (Novantrone®); nandrolone phenpropionate (Durabolin-50®); nelarabine (Arranon®); Nofetumomab (Verluma®); Oprelvekin (Neumega®); oxaliplatin (Eloxatin®); paclitaxel (Paxene®); paclitaxel (Taxol®); paclitaxel protein-bound particles (Abraxane®); palifermin (Kepivance®); pamidronate (Aredia®); pegademase (Adagen (Pegademase Bovine)®); pegaspargase (Oncaspar®); Pegfilgrastim (Neulasta®); pemetrexed disodium (Alimta®); pentostatin (Nipent®); pipobroman (Vercyte®); plicamycin, mithramycin (Mithracin®); porfimer sodium (Photofrin®); procarbazine (Matulane®); quinacrine (Atabrine®); Rasburicase (Elitek®); Rituximab (Rituxan®); sargramostim (Leukine®); Sargramostim (Prokine®); sorafenib (Nexavar®); streptozocin (Zanosar®); sunitinib maleate (Sutent®); talc (Sclerosol®); tamoxifen (Nolvadex®); temozolomide (Temodar®); teniposide, VM-26 (Vumon®); testolactone (Teslac®); thioguanine, 6-TG (Thioguanine®); thiotepa (Thioplex®); topotecan (Hycamtin®); toremifene (Fareston®); Tositumomab (Bexxar®); Tositumomab/I-131 tositumomab (Bexxar®); Trastuzumab (Herceptin®); tretinoin, ATRA (Vesanoid®); Uracil Mustard (Uracil Mustard Capsules®); valrubicin (Valstar®); vinblastine (Velban®); vincristine (Oncovin®); vinorelbine (Navelbine®); zoledronate (Zometa®) and vorinostat (Zolinza®); a pharmaceutically acceptable salt thereof, and a mixture thereof.

Any one or more of the specific dosages and dosage schedules of the compounds of the instant invention, may also be applicable to any one or more of the therapeutic agents to be used in the combination treatment (hereinafter refered to as the "second therapeutic agent").

Moreover, the specific dosage and dosage schedule of this second therapeutic agent can further vary, and the optimal dose, dosing schedule and route of administration will be determined based upon the specific second therapeutic agent that is being used.

Of course, the route of administration of the compounds of the instant invention is independent of the route of administration of the second therapeutic agent. In an embodiment, the administration for a compound of the instant invention is oral administration. In another embodiment, the administration for a compound of the instant invention is intravenous administration. Thus, in accordance with these embodiments, a compound of the instant invention is administered orally or intravenously, and the second therapeutic agent can be administered orally, parenterally, intraperitoneally, intravenously, intraarterially, transdermally, sublingually, intramuscularly, rectally, transbuccally, intranasally, liposomally, via inhalation, vaginally, intraoccularly, via local delivery by catheter or stent, subcutaneously, intraadiposally, intraarticularly, intrathecally, or in a slow release dosage form.

In addition, a compound of the instant invention and second therapeutic agent may be administered by the same mode of administration, i.e. both agents administered e.g. orally, by IV. However, it is also within the scope of the present invention to administer a compound of the instant invention by one mode of administration, e.g. oral, and to administer the second therapeutic agent by another mode of administration, e.g. IV or any other ones of the administration modes described hereinabove.

The first treatment procedure, administration of a compound of the instant invention, can take place prior to the second treatment procedure, i.e., the second therapeutic agent, after the treatment with the second therapeutic agent, at the same time as the treatment with the second therapeutic agent, or a combination thereof. For example, a total treatment period can be decided for a compound of the instant invention. The second therapeutic agent can be administered prior to onset of treatment with a compound of the instant invention or following treatment with a compound of the instant invention. In addition, anti-cancer treatment can be administered during the period of administration of a compound of the instant invention but does not need to occur over the entire treatment period of a compound of the instant invention.

The term "administration" and variants thereof (e.g., "administering" a compound) in reference to a compound of the invention means introducing the compound or a prodrug of the compound into the system of the animal in need of treatment. When a compound of the invention or prodrug thereof is provided in combination with one or more other active agents (e.g., a cytotoxic agent, etc.), "administration" and its variants are each understood to include concurrent and sequential introduction of the compound or prodrug thereof and other agents.

As used herein, the term "composition" is intended to encompass a product comprising the specified ingredients in the specified amounts, as well as any product which results, directly or indirectly, from combination of the specified ingredients in the specified amounts.

The term "therapeutically effective amount" as used herein means that amount of active compound or pharmaceutical agent that elicits the biological or medicinal response in a tissue, system, animal or human that is being sought by a researcher, veterinarian, medical doctor or other clinician.

The compounds of the instant invention are useful for the treatment and/or reducing the severity of rheumatoid arthritis.

The compounds of the instant invention are useful for the treatment and/or reducing the severity of inflammatory bowel disease.

The compounds of the instant invention are useful for the treatment and/or reducing the severity of cancer.

The compounds of the instant invention are useful for the treatment of rheumatoid arthritis.

The compounds of the instant invention are useful for the treatment of inflammatory bowel disease.

The compounds of the instant invention are useful for the treatment of cancer.

Further included within the scope of the invention is a compound of the instant invention for use in treating an inflammatory disease in a mammal.

Further included within the scope of the invention is a compound of the instant invention for use in treating an inflammatory disease in a mammal, wherein the inflammatory disease is selected from rheumatoid arthritis, inflammatory bowel disease and cancer.

Further included within the scope of the invention is a compound of the instant invention for use in treating an inflammatory disease which comprises administering a therapeutically effective amount of a compound of the instant invention in combination with a second therapeutic agent.

Further included within the scope of the invention is a compound of the instant invention for use in treating an inflammatory disease which comprises administering a therapeutically effective amount of a compound of the instant invention in combination with a second therapeutic agent, wherein the second therapeutic agent is selected from an anti-cancer agent and an anti-inflammatory agent.

Abbreviations used in the description of the chemistry and in the Examples that follow are: DCM (dichloromethane); DMF (N,N-dimethylformamide); DMSO (dimethyl sulfoxide); DMSO-d6 (dimethyl sulfoxide-d6); EtOAc (ethyl acetate); EtOH (ethanol); MS (mass spectrum); ESI (Electrospray ionization); MeOH (methanol); NMR (nuclear magnetic resonance); THF (tetrahydrofuran); TFA (trifluoroacteic acid); Boc (t-butoxycarbonyl); DMAP (4-dimethylaminopyridine); Me (methyl); TEA (triethylamine); Et (ethyl); Et₂O (diethyl ether); EDCI (1-Ethyl-3-(3-dimethylaminopropyl)carbodiimide); and SFC (Supercritical Fluid Chromatography).

### EXAMPLES

The compounds of this invention may be prepared by employing reactions as shown in the following examples, in addition to other standard manipulations that are known in the literature or exemplified in the experimental procedures.

### EXAMPLE 1

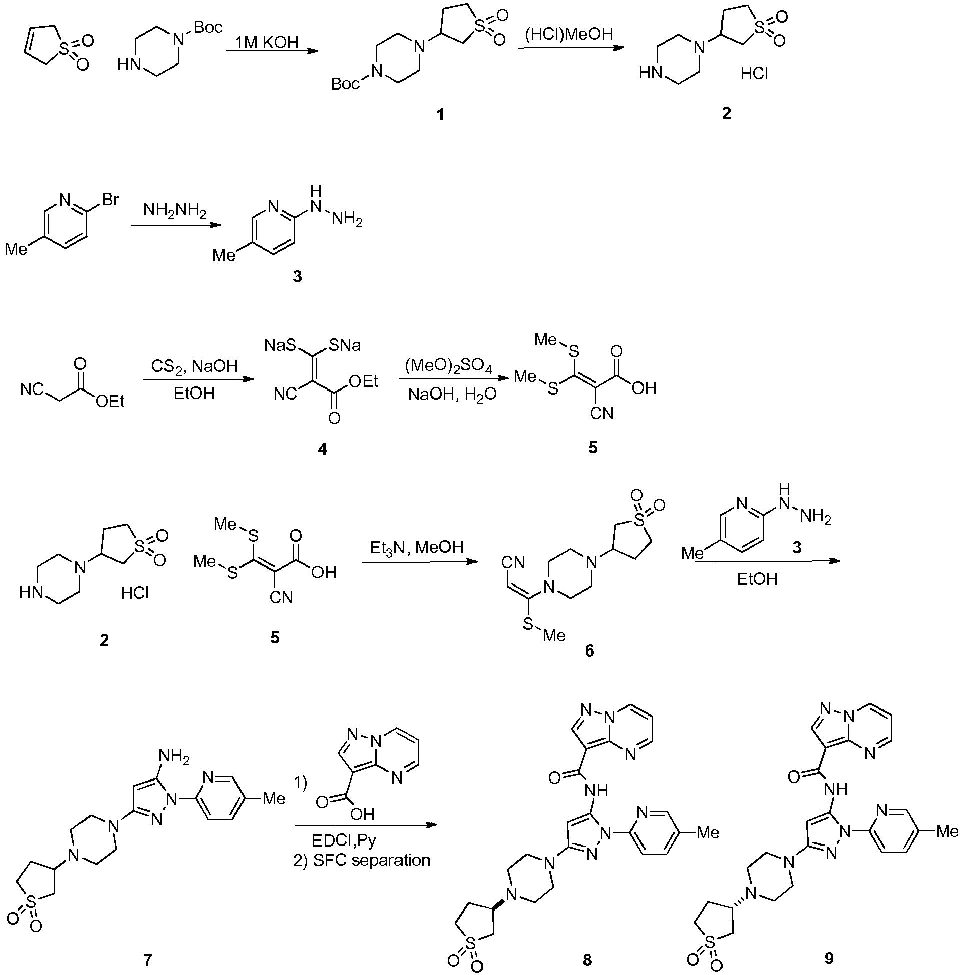

### Preparation of 1-(1,1-dioxidotetrahydrothiophen-3-yl)piperazine hydrochloride (2)

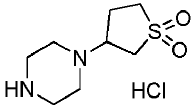

### Step 1: tert-butyl 4-(1,1-dioxidotetrahydrothiophen-3-yl)piperazine-1-carboxylate (1)

A solution of 2,5-dihydrothiophene 1,1-dioxide (574 g, 4.8 mol) and *tert*-butyl piperazine-1-carboxylate (552 g, 3 mol) in 1M KOH (9 L) was stirred at 50 °C for 12 hours. The reaction mixture was cooled to room temperature and extracted with EtOAc(8 Lx2). The combined organics were dried over Na₂SO₄, filtered, and concentrated to a crude, which was purified by column (DCM:MeOH=100:1) to give *tert*-butyl 4-(1,1-dioxidotetrahydrothiophen-3-yl)piperazine-1-carboxylate (**1**).

### Step 2: 1-(1,1-dioxidotetrahydrothiophen-3-yl)piperazine hydrochloride (2)

A solution of *tert*-butyl 4-(1,1-dioxidotetrahydrothiophen-3-yl)piperazine-1-carboxylate (**1**) (305 g, 1 mol) in 4 M HCl in MeOH(2 L) was stirred at 20 °C for 12 hours. The reaction mixture concentrated to give 1-(1,1-dioxidotetrahydrothiophen-3-yl)piperazine hydrochloride (**2**).

### Preparation of 2-hydrazinyl-5-methylpyridine (3)

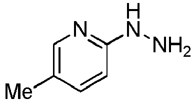

Into a 1000-mL 3-necked round-bottom flask were placed 2-bromo-5-methylpyridine (100 g, 0.58 mol) and NH₂NH₂-H₂O (400 mL). The resulting solution was stirred for 24 h at 80 °C in an oil bath. The reaction mixture was cooled to 25°C with a water bath, and then quenched by the addition of 400 mL of water. The product was precipitated by the addition of sodium chloride. The solid was collected by filtration and dried in an oven under reduced pressure to yeild 2-hydrazinyl-5-methylpyridine (**3**) as a white solid.
¹H NMR (CDCl₃, 400 MHz) δ 7.97 (s, 1H), 7.28-7.35 (d, *J* = 8.4 Hz, 1H), 6.4-6.6 (d, *J* = 8.4 Hz, 1H), 5.70 (brs. 1H), 3.72-3.74 (brs. 2H), 2.23 (m, 3H).

### N-[3-{4-[(3R)-1,1-dioxidotetrahydrothiophen-3-yl]piperazin-1-yl}-1-(5-methylpyridin-2-yl)-1H-pyrazol-5-yl]pyrazolo[1,5-a]pyrimidine-3-carboxamide and N-[3-{4-[(3S)-1,1-dioxidotetrahydrothiophen-3-yl]piperazin-1-yl}-1-(5-methylpyridin-2-yl)-1H-pyrazol-5-yl]pyrazolo[1,5-a]pyrimidine-3-carboxamide (8 and 9)

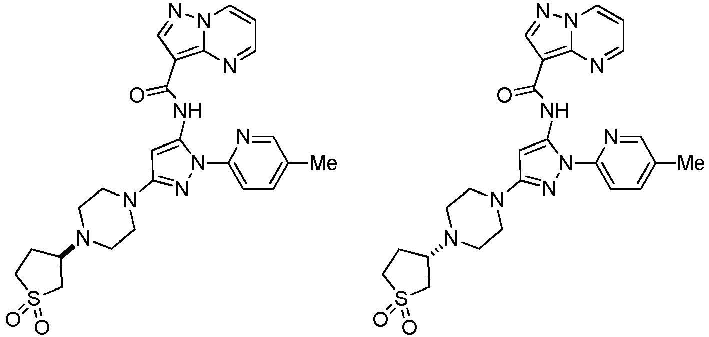

### Step 1: disodium 2-cyano-3-ethoxy-3-oxoprop-1-ene-1,1-bis(thiolate) (4)

Into a 10-L 3-necked round-bottom flask were placed ethanol (5 L), ethyl 2-cyanoacetate (800 g, 7.07 mol), and CS₂ (538 g). A solution of sodium hydroxide (566 g, 14.15 mol) in water (566 mL) was added next dropwise with stirring at 0-10 °C. When the addition was completed, the resulting solution was stirred for 15 min at 25°C and then cooled to 5 °C. The solid was collected by filtration, washed with 1.5 L of EtOH and 3 L of Et₂O. This resulted in disodium 2-cyano-3-ethoxy-3-oxoprop-1-ene-1,1-bis(thiolate) (**4**) as a yellow solid.

### Step 2: 2-cyano-3,3-bis(methylsulfanyl)prop-2-enoic acid (5)

Into a 10-L 3-necked round-bottom flask were placed sodium hydroxide (466.8 g, 11.67 mol), water (3300 mL), and ethyl 2-cyano-3-ethoxy-3-oxoprop-1-ene-1,1-bis(thiolate) (**4**) (1600 g, 6.80 mol). The resulting solution was stirred for 5 h at 40 °C in an oil bath. 5 L of ethanol was added and the layers were separated. The aqueous phase was diluted with 5 L of H₂O and cooled to 0-5°C. This was followed by the addition of dimethyl sulfate (1471 g, 11.66 mol) dropwise with stirring at 0-5 °C over 2 hr. The resulting solution was stirred for 20 min at 15 °C and an additional 20 min at 28 °C. The solid was filtered out when the mixture was cooled to 0 °C. The filtrate was adjusted to pH 2 with HCl (6 M). The solid was collected by filtration. This resulted in 2-cyano-3,3-bis(methylsulfanyl)prop-2-enoic acid (**5**) as a light brown solid.

### Step 3: (2E)-3-[4-(1,1-dioxidotetrahydrothiophen-3 -yl)piperazin-1-yl]-3-(methylsulfanyl)prop-2-enenitrile (6)

Into a 2,000-mL 3-necked round-bottom flask were placed 1-(1,1-dioxidotetrahydrothiophen-3-yl)piperazine hydrochloride (**2**) (155 g, 0.65 mol), Et₃N (457 mL, 3.2 mol) and methanol (1,500 mL). This was followed by the addition of a solution of 2-cyano-3,3-bis(methylsulfanyl)prop-2-enoic acid (**5**) (160 g, 0.84 mol) with stirring at 0 °C. The resulting solution was stirred for 12 h at 50 °C. The resulting mixture was concentrated under reduced pressure. The residue was applied onto a silica gel column and eluted with dichloromethane/methanol (100:1). This resulted in (2*E*)-3-[4-(1,1-dioxidotetrahydrothiophen-3-yl)piperazin-1-yl]-3-(methylsulfanyl)prop-2-enenitrile (**6**) as a yellow solid. MS ESI calc'd. for C₁₂H₂₀N₃O₂S₂ [M+1]⁺ 302, found 302.

### Step 4: 3-[4-(1,1-dioxidotetrahydrothiophen-3-yl)piperazin-1-yl]-1-(5-methylpyridin-2-yl)-1H-pyrazol-5-amine (7)

Into a 2000-mL 3-necked round-bottom flask were placed (2*E*)-3-[4-(1,1-dioxidotetrahydrothiophen-3-yl)piperazin-1-yl]-3-(methylsulfanyl)prop-2-enenitrile (**6**) (120 g, 0.4 mol), 2-hydrazinyl-5-methylpyridine (**3**) (73.6 g, 0.6 mol), and ethanol (1000 mL). The resulting solution was stirred for 12 h at 80 °C in an oil bath. Then it was concentrated under reduced pressure. The residue was applied onto a silica gel column and eluted with dichloromethane/methanol (100:1). This resulted in 3-[4-(1,1-dioxidotetrahydrothiophen-3-yl)piperazin-1-yl]-1-(5-methylpyridin-2-yl)-1*H*-pyrazol-5-amine (**7**) as yellow solid. MS ESI calc'd. for C₁₇H₂₅N₆O₂S [M+1]⁺ 377, found 377.

### Step 5: N-[3-{4-[(3R)-1,1-dioxidotetrahydrothiophen-3-yl]piperazin-1-yl}-1-(5-methylpyridin-2-yl)-1H-pyrazol-5-yl]pyrazolo[1,5-a]pyrimidine-3-carboxamide and N-[3-{4-[(3S)-1,1-dioxidotetrahydrothiophen-3-yl]piperazin-1-yl}-1-(5-methylpyridin-2-yl)-1H-pyrazol-5-yl]pyrazolo[1,5-a]pyrimidine-3-carboxamide (8 and 9)

A mixture of 3-[4-(1,1-dioxidotetrahydrothiophen-3-yl)piperazin-1-yl]-1-(5-methylpyridin-2-yl)-1*H*-pyrazol-5-amine (**7**) (30 g, 72.9 mmol), pyrazolo[1,5-*a*]pyrimidine-3-carboxylic acid (19 g, 120 mmol), and EDCI (30 g, 156 mmol) in pyridine (300 mL) was stirred at 60 °C for 24 hours. After cooling to room temperature, the mixture was poured into 800 mL H₂O, a solid was settled out. The suspension was filtered to give *N*-[3-{4-[(3-*rac*)-1,1-dioxidotetrahydrothiophen-3-yl]piperazin-1-yl}-1-(5-methylpyridin-2-yl)-1*H-*pyrazol-5-yl]pyrazolo[1,5-*a*]pyrimidine-3-carboxamide as a white solid, which was separated though chiral SFC (OD column 250 mm*30 mm, 10 µm, Supercritical CO_{2:}EtOH (0.05% NH₄OH) = 45:55 at 200ml/min) to afford *N*-[3-{4-[(3*R*)-1,1-dioxidotetrahydrothiophen-3-yl]piperazin-1-yl}-1-(5-methylpyridin-2-yl)-1*H-*pyrazol-5-yl]pyrazolo[1,5-*a*]pyrimidine-3-carboxamide and *N-*[3-{4-[(3*S*)-1,1-dioxidotetrahydrothiophen-3-yl]piperazin-1-yl}-1-(5-methylpyridin-2-yl)-1*H-*pyrazol-5-yl]pyrazolo[1,5-*a*]pyrimidine-3-carboxamide (**8** and **9**) as white solid. MS ESI calc'd. for C₂₄H₂₈N₉O₃S [M+1]⁺ 522, found 522. ¹H NMR (DMSO-*d6*, 400 MHz) δ 2.34-2.37 (m, 4H), 2.49-2.50 (m, 4H), 2.50-2.56 (m, 3H), 2.99-3.37 (m, 8H),6.67 (s, 1H), 7.36-7.37 (dd, *J*= 4.5, 7.2Hz, 1H),7.68 (d, *J*= 8.7Hz, 1H),7.77 (d, *J*= 6.3Hz, 1H),8.45 (s,1H),8.73 (s, 1H), 9.14-9.16 (dd, *J*= 1.5, 3.9Hz, 1H),9.38-9.40 (dd, *J*= 1.5, 6.9Hz, 1H), 13.27 (s, 1H).

### EXAMPLE 2

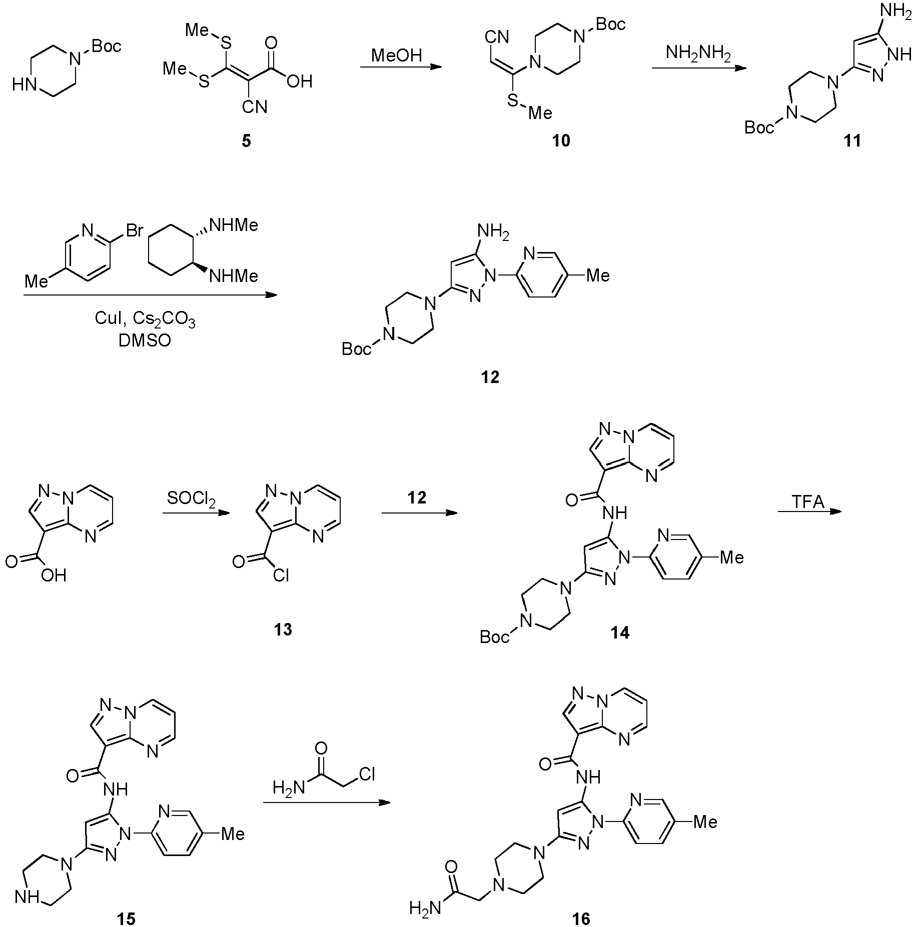

### Preparation of tert-butyl 4-[5-amino-1-(5-methylpyridin-2-yl)-1H-pyrazol-3-yl]piperazine-1-carboxylate (12)

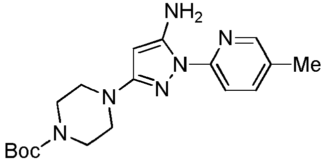

### Step 1: tert-butyl 4-[(E)-2-cyano-1-(methylsulfanyl)ethenyl]piperazine-1-carboxylate (10)

Into a 10-L 3-necked round-bottom flask were placed 2-cyano-3,3-bis(methylsulfanyl)prop-2-enoic acid (**5**) (450 g, 2.38 mol), methanol (6 L), and *tert*-butyl piperazine-1-carboxylate (1,781 g, 9.56 mol). The resulting solution was stirred overnight at 25 °C. The resulting mixture was concentrated under vacuum and the residue was used in the next step directly.

### Step 2: tert-butyl 4-(5-amino-1H-pyrazol-3-yl)piperazine-1-carboxylate (11)

Into a 10-L 3-necked round-bottom flask were placed *tert*-butyl 4-[(*E*)-2-cyano-1-(methylsulfanyl)ethenyl]piperazine-1-carboxylate (**10**) (674 g), NH₂NH₂.H₂O (357 g), and ethanol (5 L). The resulting solution was stirred overnight at 80 °C in an oil bath. The reaction mixture was cooled and concentrated under reduced pressure. The residue was applied onto a silica gel column and eluted with ethyl acetate/petroleum ether (1:1). This resulted in *tert*-butyl 4-(5-amino-1*H*-pyrazol-3-yl)piperazine-1-carboxylate (**11**) as a light brown solid.

### Step 3: tert-butyl 4-[5-amino-1-(5-methylpyridin-2-yl)-1H-pyrazol-3-yl]piperazine-1-carboxylate (12)

Into a 3-L 3-necked round-bottom flask purged and maintained with an inert atmosphere of nitrogen were placed *tert*-butyl 4-(5-amino-1*H-*pyrazol-3-yl)piperazine-1-carboxylate (**11**) (144 g, 539 mmol), DMSO (1500 mL), 2-bromo-5-methylpyridine (97.4 g, 569 mmol), CuI (10.26 g, 53.9 mmol), Cs₂CO₃ (352 g), and (1*S*,2*S*)-1-N,2-N-dimethylcyclohexane-1,2-diamine (7.7 g, 54.13 mmol). The resulting solution was stirred for 3.5 h at 130 °C in an oil bath. The reaction mixture was cooled and then quenched by the addition of 1.5 L of water. The resulting solution was extracted with ethyl acetate (1.5 L x 3). The organic layers were combined, washed with H₂O (1.5 L x 3), and dried over anhydrous sodium sulfate. The residue was applied onto a silica gel column and eluted with ethyl acetate/petroleum ether (1:10). The collected fractions were combined and concentrated under reduced pressure. This resulted in *tert*-butyl 4-[5-amino-1-(5-methylpyridin-2-yl)-1*H-*pyrazol-3-yl]piperazine-1-carboxylate (**12**) as a purple solid. MS ESI calc'd. for C₁₈H₂₇N₆O₂ [M+1]⁺ 359, found 359. ¹H NMR (DMSO-*d6*, 400 MHz) δ 8.13 (1H, s), 7.67-7.69 (1H, m), 7.57-7.59 (1H, d), 6.77 (2H, s), 5.02 (1H, s), 3.33-3.42 (4H, m), 3.09-3.12 (4H, m), 2.27 (3H, s), 1.42 (9H, s).

### N-{3-[4-(2-amino-2-oxoethyl)piperazin-1-yl]-1-(5-methylpyridin-2-yl)-1H-pyrazol-5-yl}pyrazolo[1,5-a]pyrimidine-3-carboxamide (16)

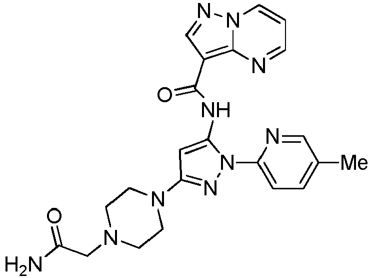

### Step 1: pyrazolo[1,5-a]pyrimidine-3-carbonyl chloride (13)

To a solution of pyrazolo[1,5-*a*]pyrimidine-3-carboxylic acid (23.0 g, 0.14 mol) in thionyl chloride (118 g, 0.99 mol) was added DMF (0.3 mL). The reaction was heated to 80 °C and stirred at this temperature for 2 hours. Hexanes were added at 50 °C. The mixture was filtered and the solid was washed with hexanes, dried to give pyrazolo[1,5-*a*]pyrimidine-3-carbonyl chloride (**13**) as a yellow solid.

### Step 2: tert-butyl 4-{1-(5-methylpyridin-2-yl)-5-[(pyrazolo[1,5-a]pyrimidin-3-ylcarbonyl)amino]-1H-pyrazol-3-yl}piperazine-1-carboxylate (14)

To a solution of pyrazolo[1,5-*a*]pyrimidine-3-carbonyl chloride (**13**) (28.0 g, 0.16 mol) in THF (400 mL) were added *tert*-butyl 4-[5-amino-1-(5-methylpyridin-2-yl)-1*H-*pyrazol-3-yl]piperazine-1-carboxylate (**12**) (37.0 g, 0.10 mol), TEA (31.0 g, 0.31 mol) and DMAP (6.0 g, 0.05 mol) sequentially at room temperature. The reaction mixture was sirred at 55 °C for 4 h. The reaction was cooled to room temperature, then treated with water. The mixture was filtered, and the solid was dried to give *tert*-butyl 4-{1-(5-methylpyridin-2-yl)-5-[(pyrazolo[1,5-*a*]pyrimidin-3-ylcarbonyl)amino]-1*H-*pyrazol-3-yl}piperazine-1-carboxylate (**14**) as a yellow solid.

### Step 3-4: N-{3-[4-(2-amino-2-oxoethyl)piperazin-1-yl]-1-(5-methylpyridin-2-yl)-1H-pyrazol-5-yl}pyrazolo[1,5-a]pyrimidine-3-carboxamide (16)

To a solution of *tert*-butyl 4-{1-(5-methylpyridin-2-yl)-5-[(pyrazolo[1,5-*a*]pyrimidin-3-ylcarbonyl)amino]-1*H-*pyrazol-3-yl}piperazine-1-carboxylate (**14**) (45.0 g, 0.089 mol) in DCM (500 mL) was added TFA (122.0 g, 1.07 mol) slowly. The reaction was stirred at room temperature for 3 h. The reaction mixture was concentrated under reduced pressure to give *N*-[1-(5-methylpyridin-2-yl)-3-(piperazin-1-yl)-1*H*-pyrazol-5-yl]pyrazolo[1,5-*a*]pyrimidine-3-carboxamide (**15**) which was used next step directly without further purification.

A solution of *N*-[1-(5-methylpyridin-2-yl)-3-(piperazin-1-yl)-1*H-*pyrazol-5-yl]pyrazolo[1,5-*a*]pyrimidine-3-carboxamide (**15**) in DMF (500 mL) were added K₂CO₃ (86.0 g, 0.62 mol) and 2-chloroacetamide (16.5 g, 0.18 mol) sequentially at room temperature. The reaction was stirred at 50 °C for 1.5 h, then poured into water and filtered. The solid was dried to afford *N*-{3-[4-(2-amino-2-oxoethyl)piperazin-1-yl]-1-(5-methylpyridin-2-yl)-1*H-*pyrazol-5-yl}pyrazolo[1,5-*a*]pyrimidine-3-carboxamide (**16**) as a yellow solid. MS ESI calc'd. for C₂₂H₂₅N₁₀O₂ [M+1]⁺ 461, found 461. ¹H NMR (CDCl₃, 300 MHz) δ 2.37 (s, 3H), 2.71 (t, 4H), 3.08 (s, 2H), 3.40 (t, 4H), 5.49 (br s, 1H), 6.75 (s, 1H), 7.08 (dd, 2H), 7.61 (d, 1H), 7.78 (d, 2H), 8.23 (d, 1H), 8.78 (s, 1H), 8.81-8.85 (m, 2H), 13.35 (s, 1H).

### BIOLOGICAL DATA

### Determination of IRAK4 Kinase Activity

The kinase activity of IRAK4 is determined by its ability to catalyze the phosphorylation of a fluorescent polypeptide substrate. The extent of phosphorylation is measured using IMAP technology (Molecular Devices) where the phosphorylated fluorescent substrate binds to the large M(III)-based nanoparticles which reduces the rotational speed of the substrate and thus increases its fluorescent polarization (FP).

Procedure: A 20 µl reaction mixture contains 10 mM TriHCl, pH 7.2, 1 nM GST tagged IRAK4 (SignalChem), 100 nM fluorescent peptide substrate (RP7030, Molecular Devices), 100 µM ATP, 1 mM DTT, 2 mM MgCl₂, and 0.01% Tween 20. The reaction is initiated by the addition of ATP. After incubation for 1 hour at 25 °C, 60 µl of Progressive IMAP Reagent (Molecular Devices) is added to stop the reaction. Change in RP7030's FP is determined by a FP reader (EnVision Multilabel Reader, Perkin Elmer).

### Pharmacokinetic Properties

Pharmacokinetic parameters were determined following PO and SC dosing at 30 mg/kg in fed female Lewis rats. Compounds were formulated for both PO and SC dosing in 50/50 (v/v) 50% captisol/50 mM citrate buffer (pH 4). Plasma samples obtained from dosed animals were prepared for analysis by means of a single step protein precipitation technique by adding 200 µL of acetonitrile to 50 µL aliquots of individual subject samples. Samples were mixed by vortex for homogeneity and then subjected to centrifugation at 3500 rpm for 10 min. The supernatant (200 µL) was collected and injected into the LC-MS/MS for analysis. Pharmacokinetic parameters were calculated using established non-compartmental methods. Maximum concentrations are summarized in the table below (Table 1).

As shown in Table 1, Compounds **8, 9,** and **16** of the instant invention displayed superior Cmax at either 30 mpk SC dosing or 30 mpk PO dosing or both in rats compared to that of the Compound **A.** Compound **A** is disclosed in WO2012/129258.

**Table 1: Summary of Cmax after PO or SC dosing**

| | | | | |
|---|---|---|---|---|
| | 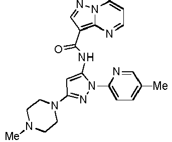 | 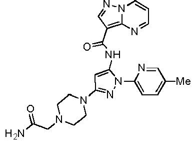 | 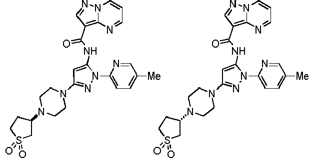 | |

| Compound | **A** | **16** | **8** | **9** |
|---|---|---|---|---|
| | | | fast eluting | slow eluting |
| IRAK4 IMAP IC₅₀ (nM) | 2 | 4 | 1 | 2 |
| Rat PK, 30 mpk SC, Cmax (µM) | 3.3 | 16.1 | 1.5 | 8.1 |
| Rat PK, 30 mpk PO, Cmax (µM) | 1.0 | 4.3 | 8.6 | 7.7 |

## Claims

1. A compound which is selected from:
*N*-[3-{4-[(3*R*)-1,1-dioxidotetrahydrothiophen-3-yl]piperazin-1-yl}-1-(5-methylpyridin-2-yl)-1*H*-pyrazol-5-yl]pyrazolo[1,5-*a*]pyrimidine-3-carboxamide;
*N*-[3-{4-[(3*S*)-1,1-dioxidotetrahydrothiophen-3-yl]piperazin-1-yl}-1-(5-methylpyridin-2-yl)-1*H*-pyrazol-5-yl]pyrazolo[1,5-*a*]pyrimidine-3-carboxamide; and
*N*-{3-[4-(2-amino-2-oxoethyl)piperazin-1-yl]-1-(5-methylpyridin-2-yl)-1*H*-pyrazol-5-yl}pyrazolo[1,5-*a*]pyrimidine-3-carboxamide;
or a pharmaceutically acceptable salt or stereoisomer thereof.

2. The compound according to Claim 1 for use in the treatment or prevention of an inflammation related disease in a mammal.

3. The compound for use according to Claim 2 wherein the inflammatory disease is selected from rheumatoid arthritis, inflammatory bowel disease and cancer.

4. The compound for use according to claim 2 which comprises administering a therapeutically effective amount of a compound of Claim 1 in combination with a second therapeutic agent.

5. The compound for use of Claim 4 wherein the second therapeutic agent is selected from an anti-cancer agent and an anti-inflammatory agent.

## Patentansprüche

1. Eine Verbindung, die ausgewählt ist aus:
*N*-[3-{4-[(3*R*)-1,1-Dioxidotetrahydrothiophen-3-yl]piperazin-1-yl}-1-(5-methylpyridin-2-yl)-1*H*-pyrazol-5-yl]pyrazolo[1,5-*a*]pyrimidin-3-carboxamid,
*N*-[3-{4-[(3*S*)-1,1-Dioxidotetrahydrothiophen-3-yl]piperazin-1-yl}-1-(5-methylpyridin-2-yl)-1*H*-pyrazol-5-yl]pyrazolo[1,5-*a*]pyrimidin-3-carboxamid und
*N*-{3-[4-(2-Amino-2-oxoethyl)piperazin-1-yl]-1-(5-methylpyridin-2-yl)-1*H*-pyrazol-5-yl}pyrazolo[1,5-a]pyrimidin-3-carboxamid,
oder ein pharmazeutisch annehmbares Salz oder Stereoisomer davon.

2. Die Verbindung gemäß Anspruch 1 zur Verwendung bei der Behandlung oder Prävention einer mit einer Entzündung zusammenhängenden Erkrankung bei einem Säuger.

3. Die Verbindung zur Verwendung gemäß Anspruch 2, wobei die Entzündungserkrankung ausgewählt ist aus rheumatoider Arthritis, entzündlicher Darmerkrankung und Krebs.

4. Die Verbindung zur Verwendung gemäß Anspruch 2, die die Verabreichung einer therapeutisch wirksamen Menge einer Verbindung nach Anspruch 1 in Kombination mit einem zweiten therapeutischen Mittel umfasst.

5. Die Verbindung zur Verwendung nach Anspruch 4, wobei das zweite therapeutische Mittel ausgewählt ist aus einem Mittel gegen Krebs und einem entzündungshemmenden Mittel.

## Revendications

1. Composé qui est sélectionné parmi :
*N*-[3-{4-[(3R)-1,1-dioxidotétrahydrothiophène-3-yle]pipérazine-1-yle}-1-(5-méthylpyridine-2-yle)-1H-pyrazol-5-yle]pyrazolo[1,5-*a*]pyrimidine-3-carboxamide ;
N-[3-{4-[(3S)-1,1-dioxidotétrahydrothiophène-3-yle]pipérazine-1-yle}-1-(5-méthylpyridine-2-yle)-1H-pyrazol-5-yle]pyrazolo[1,5-a]pyrimidine-3-carboxamide ; et
N-{3-[4-(2-amino-2-oxoéthyle)pipérazine-1-yle]-1-(5-méthylpyridine-2-yle)-1H-pyrazol-5-yle}pyrazolo[1,5-a]pyrimidine-3-carboxamide ;
ou un sel ou stéréoisomère pharmaceutiquement acceptable de celui-ci.

2. Composé selon la revendication 1 pour une utilisation dans le traitement ou de la prévention d'une maladie liée à une inflammation chez un mammifère.

3. Composé pour une utilisation selon la revendication 2, dans laquelle la maladie inflammatoire est sélectionnée parmi l'arthrite rhumatoïde, la maladie inflammatoire de l'intestin et le cancer.

4. Composé pour une utilisation selon la revendication 2, comprenant l'administration d'une quantité efficace thérapeutiquement d'un composé selon la revendication 1 en combinaison avec un second agent thérapeutique.

5. Composé pour une utilisation selon la revendication 4, dans laquelle le second agent thérapeutique est sélectionné parmi un agent anticancéreux et un agent anti-inflammatoire.
